# EUROPEAN PATENT SPECIFICATION

(11) **EP 1 845 300 B1**
(45) Date of publication and mention of the grant of the patent: **10.08.2011**
(21) Application number: 07105412.6
(22) Date of filing: 30.03.2007
(51) Int. Cl.: F16L 55/11

(54) **Closure plug**
Verschlußstopfen
Bouchon de fermeture

(30) Priority: 10.04.2006 GB 0607159
(43) Date of publication of application: 17.10.2007
(73) Proprietor: The BOC Group Limited, The Surrey Research Park Guildford Surrey GU2 7XY (GB)
(72) Inventor: Maycock, Richard Philip, Sheffield, S20 3RP (GB)
(74) Representative: Wickham, Michael

(56) References cited:
- WO-A-87/01008
- WO-A-99/18383
- DE-A1- 2 705 319
- US-A- 5 236 005
- US-A- 5 562 121

## Description

This invention relates to a dummy closure plug for a medical gas terminal unit.

By the term "medical gas terminal unit" is meant a terminal unit for a medical gas pipeline the terminal unit comprising (a) a socket having a central passageway for the insertion a probe which can be coupled to equipment to be placed in gas flow communication with the medical gas pipeline, (b) a locking mechanism which is able to co-operate with a detent in the probe to lock the probe in position, (c) an outer release collar which is able to be urged against the bias of a spring to unlock the probe and (d) a check valve able to be opened by insertion of the probe in the socket.

In one commonly-used medical gas terminal unit, the socket has a pair of inclined slots in which a plurality of bars is located, the bars being supported on a platform that is urged forwards by a helical compression spring. When the prove 8 is inserted into the passageway of the socket, its tip eventually abuts the bars, depresses them against the bias of the compression spring and they move downwards and outwards along ramped edges of the slots allowing the probe to move further into the passageway. The probe is pushed into the passageway until the bars reach the detent, typically a circumferential recess, at which point the spring uses them into the recess to clamp the probe in position. The probe is typically hollow to permit delivery of gas therethrough. Removal of the probe is achieved by depressing an outer collar which depresses the platforms and permits the bars to move outwardly from the recess so that the probe can be withdrawn from the passageway. Such an arrangement is described in WO-A-87101008 with reference to its Figure 1.

The terminal unit is typically fitted with both a check valve and a separate maintenance valve. Insulation of the probe into the socket opens the check valve, the forward end of the probe being able to displace a valve member of the check valve from a seat so as to open the valve.

From time to time, a need arises to take a medical gas pipeline out of use for routine cleaning, maintenance or repair. It is important that the hospital staff know that the pipeline is out of use. Current practice is to stick to the medical gas terminal unit a label bearing the instruction "Do not use". Some labels are simply of a size that they do not obstruct connection of medical equipment to the terminal unit. Other labels although covering most or all of the socket can simply be manually unpeeled and may carry with them the terminal unit identification label. Neither kind of label is effective in preventing the inadvertent connection of medical equipment to a medical gas pipeline that is out of use. Such inadvertent connection is hazardous to health, either because the pipeline is unable to deliver gas to a patient or because maintenance or repair procedures have resulted in the pipeline holding temporarily a different gas from the one it usually delivers.

It is an aim of the invention to provide a solution to the above-described problem.

According to the present invention there is provided A dummy closure plug for a medical gas terminal unit of a medical pipeline, the medical gas terminal unit comprising, (i) a socket having a central passageway for the insertion of a probe which can be coupled to equipment to be placed in gas flow communication with the medical gas pipeline, (ii) a locking mechanism which is able to co-operate with a detent in the probe to lock the probe in position, (iii) an outer release collar which is able to be urged against the bias of a spring to unlock the probe and (iv) a check valve able to be opened by insertion of the probe in the socket, the dummy closure plug comprising (a) a spigot adapted to be locked in position by the locking mechanism of the medical gas terminal unit when the plug is inserted in the socket of the medical gas terminal unit, the spigot being of such a length that when the dummy closure plug is in a locked position in the terminal unit, the spigot does not reach the terminal unit check valve, and (b) a body from which the spigot projects and which, when the closure plug is in locking engagement with the socket, obstructs access to the release collar of the medical gas terminal unit, wherein the body has at least one aperture therethrough for the insertion of an unlocking tool to urge the release collar to release the dummy closure plug, the dummy closure plug, when locked in the unit, preventing the connection of the equipment to the medical gas terminal unit.

Accordingly, when a medical gas pipeline is out of use, a dummy closure plug according to the invention can be inserted in the pipeline's terminal unit. The plug, if properly inserted, cannot be removed without the aid of an unlocking tool to which hospital medical staff will not normally have access. Therefore, the risk of inadvertent connection of medical equipment to an out-of-use medical gas pipeline is eliminated.

Preferably, the body of the dummy closure plug has a pair of release apertures therethrough so as to enable a release pressure to be applied to the release collar of the medical gas terminal units at two different points.

Preferably, the body has a face bearing indicia that convey appropriate information to medical staff. For example, the face may bear the legend, "DO NOT USE".

Preferably, the spigot has a detent in the form of a circumferential recess.

A dummy closure plug according to the invention will now be described by way of example with reference to the accompanying drawings, in which:
Figure 1 is a schema of a medical gas terminal unit;
Figure 2 is a side elevation of a dummy closure plug according to the invention;
Figure 3 is a front view of the dummy closure plug shown in Figure 2, and
Figure 4 is a plan view of an unlocking tool which is able to effect unlocking of the closure plug shown in Figures 2 and 3 from the medical gas terminal unit shown in Figure 1.

The drawings are not to scale.

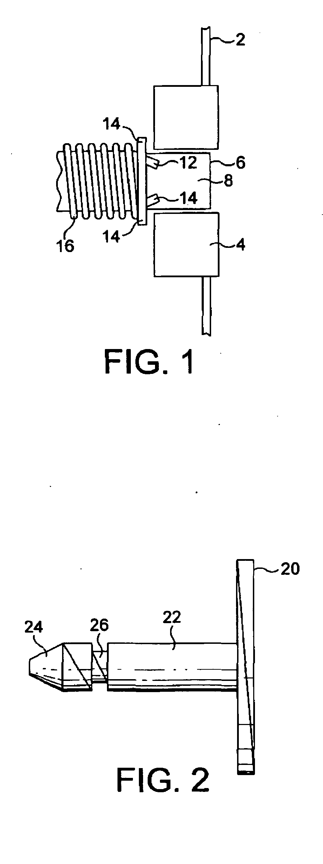

Referring to Figure 1 of the drawings, the medical gas terminal unit comprises a wall plate 2 which retains by means not shown but well known in the art an outer release collar 4. The release collar 4 surrounds a socket 6 having a central passageway 8 therethrough. The socket 6 has a pair of inclined slots 10 in which bars 12 or other gripping members are located. The bars 12 are supported on a platform 14 that is urged outward by a helical compression spring 16.

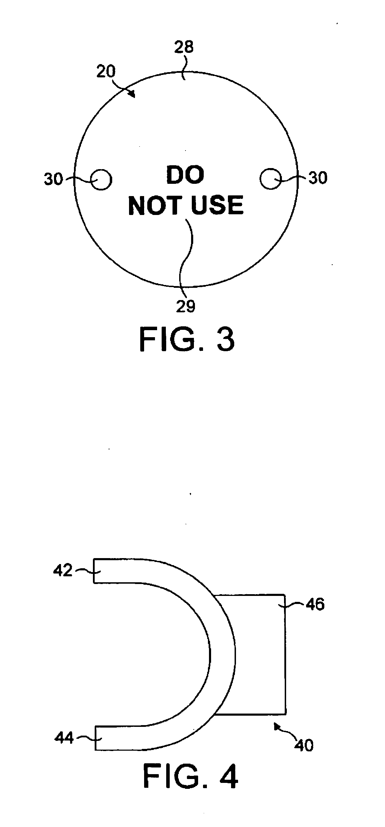

Referring now to Figures 2 and 3 of the drawings, the dummy closure plug comprises a body 20 and a central spigot 22, the spigot 22 extending perpendicularly to the body 20. Typically, the spigot 22 is integral with the body 20. The spigot 22 terminates in an integral nose 24 that tapers towards its distal end. The spigot 22 also has a detent 26 in the form of a circumferential recess. With reference particularly to Figure 3, the body 20 has a face 28 carrying suitable warning indicia 29, for example, the words "DO NOT USE". The body 20 also has a pair of apertures 30 formed therethrough.

The spigot 22 of the dummy closure plug is complementary to the passageway 8 of the socket 6 of the medical gas terminal unit shown in Figure 1. When the medical gas pipeline (not shown) that terminates in the terminal unit shown in Figure 1 is out of use the dummy plug can be inserted. When the spigot 22 is inserted in the socket 6, its nose 24 eventually abuts the bars 12, depresses them against the bias of the compression spring 16 and they move outwards along the edges of the slots 10 allowing the spigot 22 to move further into the passageway 8. The spigot 22 is pushed further into the passageway 8 until the bars reach the detent 26 at which point the compression spring 16 urges them into the recess to clamp (lock) the spigot 22 in position. The spigot 22 cannot then be pushed any further distance into the passageway 8. The length of the spigot 22 is such that when it is in its locked position its nose terminates short of a check valve (not shown) that forms part of the medical gas terminal unit. The medical gas pipeline thus remains closed. The length of the spigot 22 is also such that then it is in its locked position, the body 20 of the dummy closure plug is positioned adjacent to the release collar 4 of the medical gas terminal unit 2. The body 20 is of a size such that, in this position, it effectively blocks manual access to the release collar 4. Thus, removal of the plug cannot be effected by pressing the hand on the release collar 4.

Instead, a release tool such as that shown in Figure 4 needs to be used. Referring to Figure 4, the release tool 40 has prongs 42 and 44 and a handle 46. Manual insertion of the prongs 42 and 44 through the apertures 30 in the body 20 of the dummy closure plug causes them to bear against the outer release collar 4 of the medical gas terminal unit. The pressure that the tool 40 applies to the release collar 4 causes it to move inwardly so that it bears against the platform 14. Continued pressure exerted through the tool 40 causes the platform 14 to be displaced inwards against the bias of the compression spring 16. This action permits the bars 12 to move outwardly from the detent 26 so that the dummy closure plug can be withdrawn from the socket 6.

The dummy closure plug is preferably formed as a one-piece moulding from a suitable plastics material, which is preferably non-oil leaching. The plastics material should also permit printing of the indicia 29 on its face 28. The body 20 need not be of any great thickness. It may for example be only 2 to 3mm thick. The indicia 29 may be raised by a further millimetre.

The dummy closure plug of the invention cannot fall out of the medical gas terminal unit and is effective to prevent unauthorised coupling of equipment to the terminal unit when its medical gas pipeline is out of service.

## Claims

1. A dummy closure plug for a medical gas terminal unit of a medical pipeline, the medical gas terminal unit comprising, (i) a socket (6) having a central passageway (8) for the insertion of a probe which can be coupled to equipment to be placed in gas flow communication with the medical gas pipeline, (ii) a locking mechanism (12, 14, 16) which is able to co-operate with a detent in the probe to lock the probe in position, (iii) an outer release collar (14) which is able to be urged against the bias of a spring (16) to unlock the probe and (iv) a check valve able to be opened by insertion of the probe in the socket (6), the dummy closure plug comprising (a) a spigot (22) adapted to be locked in position by the locking mechanism (13, 14, 16) of the medical gas terminal unit when the plug is inserted in the socket (6) of the medical gas terminal unit, the spigot (22) being of such a length that when the dummy closure plug is in a locked position in the terminal unit, the spigot (22) does not reach the terminal unit check valve, and (b) a body (20) from which the spigot (22) projects and which, when the closure plug is in locking engagement with the socket, obstructs access to the release collar (4) of the medical gas terminal unit, wherein the body (20) has at least one aperture (30) therethrough for the insertion of an unlocking tool to urge the release collar (4) to release the dummy closure plug, the dummy closure plug, when locked in the unit, preventing the connection of the equipment to the medical gas terminal unit.

2. A dummy closure plug as claimed in claim 1, where in the dummy closure plug has a pair of release apertures (30) therethrough.

3. A dummy closure plug as claimed in claim 1 or claim 2, wherein the body (6) has a face (28) bearing indicia (29).

4. A dummy closure plug as claimed in any one of the preceding clams, wherein the spigot (22) has a detent (26)in the form of a circumferential recess.

5. A dummy closure plug as claimed in any one of the preceding claims, when inserted in a medical gas terminal unit.

## Patentansprüche

1. Blindverschlussstopfen für eine medizinische Gasanschlusseinheit einer medizinischen Rohrleistung, wobei die medizinische Gasanschlusseinheit Folgendes umfasst: (i) eine Buchse (6) mit einem mittleren Durchlass (8) für das Einführen einer Sonde, die mit einer Einrichtung gekoppelt werden kann, welche in Gasströmungsverbindung mit der medizinischen Gasrohrleitung platziert werden soll, (ii) einen Verriegelungsmechanismus (12, 14, 16), der mit einer Arretierung in der Sonde zusammenwirken kann, um die Sonde in Position zu verriegeln, (iii) einen äußeren Freigabebund (14), der gegen die Vorspannung einer Feder (16) gedrückt werden kann, um die Sonde zu entriegeln, und (iv) ein Rückschlagventil, das durch Einführen der Sonde in die Buchse (6) geöffnet werden kann, wobei der Blindverschlussstopfen (a) einen Zapfen (22), der zur Verriegelung in Position durch den Verriegelungsmechanismus (12, 14, 16) der medizinischen Gasanschlusseinheit, wenn der Stopfen in der Buchse (6) der medizinischen Gasanschlusseinheit eingeführt ist, ausgeführt ist, wobei der Zapfen (22) eine solche Länge aufweist, dass, wenn sich der Blindverschlussstopfen in einer verriegelten Position in der Anschlusseinheit befindet, der Zapfen (22) das Anschlusseinheitsrückschlagventil nicht erreicht, und (b) einen Körper (20), von dem der Zapfen (22) vorragt und der, wenn sich der Verschlussstopfen in Verriegelungseingriff mit der Buchse befindet, Zugang zu dem Freigabebund (4) der medizinischen Gasanschlusseinheit blockiert, enthält, wobei in dem Körper (20) mindestens eine Öffnung (30) zum Einführen eines Entriegelungswerkzeugs zwecks Drückens des Freigabebunds (4) zur Freigabe des Blindverschlussstopfens ausgebildet ist, wobei der Blindverschlussstopfen, wenn er in der Einheit verriegelt ist, die Verbindung der Einrichtung mit der medizinischen Gasanschlusseinheit verhindert.

2. Blindverschlussstopfen nach Anspruch 1, wobei Blindverschlussstopfen ein Paar Freigabeöffnungen (30) ausgebildet ist.

3. Blindverschlussstopfen nach Anspruch 1 oder 2, wobei der Körper (6) eine Angaben (29) tragende Fläche (28) aufweist.

4. Blindverschlussstopfen nach einem der vorhergehenden Ansprüche, wobei der Zapfen (22) eine Arretierung (26) in Form einer Umfangsaussparung aufweist.

5. Blindverschlussstopfen nach einem der vorhergehenden Ansprüche, wenn er in einer medizinischen Gasanschlusseinheit eingeführt ist.

## Revendications

1. Bouchon fictif de fermeture destiné à une unité de terminaison de gaz médical d'une canalisation à usage médical, l'unité de terminaison de gaz médical comportant, (i) une douille (6) présentant un passage central (8) pour l'introduction d'une sonde qui peut être couplée à un équipement devant être placé en communication d'écoulement gazeux avec la canalisation de gaz médical, (ii) un mécanisme de verrouillage (12, 14, 16) qui peut coopérer avec une détente dans la sonde pour verrouiller la sonde en place, (iii) un collier externe de libération (14) qui peut être forcé contre la sollicitation d'un ressort (16) pour déverrouiller la sonde et (iv) et un clapet antiretour pouvant être ouvert par l'introduction de la sonde dans la douille (6), le bouchon fictif de fermeture comportant (a) un ergot (22) conçu pour être verrouillé en place par le mécanisme de verrouillage (13, 14, 16) de l'unité de terminaison de gaz médical lorsque le bouchon est introduit dans la douille (6) de l'unité de terminaison de gaz médical, l'ergot (22) ayant une longueur telle que lorsque le bouchon fictif de fermeture est dans une position verrouillée dans l'unité de terminaison, l'ergot (22) n'atteint pas le clapet antiretour de l'unité de terminaison, et (b) un corps (20) d'où sort l'ergot (22) et qui, lorsque le bouchon de fermeture est engrené et verrouillé avec la douille, interdit l'accès au collier de libération (4) de l'unité de terminaison de gaz médical, dans lequel le corps (20) présente au moins une ouverture (30) pour l'introduction d'un outil de déverrouillage afin de forcer le collier de libération (4) pour qu'il libère le bouchon fictif de fermeture, le bouchon fictif de fermeture, lorsque verrouillé dans l'unité, empêchant le raccordement de l'équipement à l'unité de terminaison de gaz médical.

2. Bouchon fictif de fermeture selon la revendication 1, dans lequel le bouchon fictif de fermeture présente une paire d'ouvertures de libération (30).

3. Bouchon fictif de fermeture selon la revendication 1 ou la revendication 2, dans lequel le corps (6) a une face (28) portant des indices (29).

4. Bouchon fictif de fermeture selon l'une quelconque des revendications précédentes, dans lequel l'ergot (22) est pourvu d'une détente (26) sous la forme d'un renfoncement circonférentiel.

5. Bouchon fictif de fermeture selon l'une quelconque des revendications précédentes, lorsqu'il est introduit dans une unité de terminaison de gaz médical.
